(19) Europäisches Patentamt
European Patent Office
Office européen des brevets

(11) Veröffentlichungsnummer: **0 175 199**
**A2**

(12) **EUROPÄISCHE PATENTANMELDUNG**

(21) Anmeldenummer: **85111021.3**

(22) Anmeldetag: **02.09.85**

(51) Int. Cl.[4]: **C 07 F 7/08**, A 01 N 55/00

(30) Priorität: **12.09.84 DE 3433390**

(43) Veröffentlichungstag der Anmeldung: **26.03.86**
**Patentblatt 86/13**

(84) Benannte Vertragsstaaten: **AT BE CH DE FR GB IT LI NL SE**

(71) Anmelder: **BAYER AG, Konzernverwaltung RP**
**Patentabteilung, D-5090 Leverkusen 1 Bayerwerk (DE)**

(72) Erfinder: **Förster, Heinz, Dr., Am Eckbusch 47,**
**D-5600 Wuppertal 1 (DE)**
Erfinder: **Eue, Ludwig, Dr., Paul-Klee-Strasse 36,**
**D-5090 Leverkussen 1 (DE)**
Erfinder: **Santel, Hans-Joachim, Dr., Gerstenkamp 19,**
**D-5000 Köln 80 (DE)**
Erfinder: **Schmidt, Robert R., Dr., Im Waldwinkel 110,**
**D-5060 Bergisch-Gladbach 2 (DE)**

(54) **Phenoxypropionsäure-Derivate.**

(57) Neue Phenoxypropionsäure-Derivate der Formel

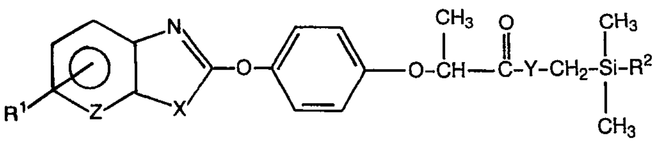

(I)

in welcher
X für Sauerstoff oder Schwefel steht,
Y für Sauerstoff, Schwefel oder die Gruppierung der Formel NR steht, wobei R für Wasserstoff oder Alkyl mit 1 bis 6 Kohlenstoffatomen steht,
Z für Stickstoff oder die CH-Gruppe steht,
$R^1$ für Wasserstoff oder Halogen steht und
$R^2$ für Alkyl mit 1 bis 6 Kohlenstoffatomen steht,
mehrere Verfahren zur Herstellung der neuen Wirkstoffe und deren Verwendung als Herbizide.

Neue Zwischenprodukte der Formel

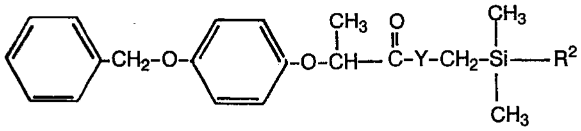

(XII)

in welcher
Y und $R^2$ die oben angegebene Bedeutung haben, sowie ein Verfahren zur Herstellung dieser Stoffe.

0175199

BAYER AKTIENGESELLSCHAFT 5090 Leverkusen, Bayerwerk

Konzernverwaltung RP
Patentabteilung KM/ABc
Ib

Phenoxypropionsäure-Derivate

Die Erfindung betrifft neue Phenoxypropionsäure-Derivate, mehrere Verfahren zu deren Herstellung und ihre Verwendung als Herbizide.

Es ist bereits bekannt, daß zahlreiche Phenoxypropionsäure-Derivate herbizide Eigenschaften besitzen (vgl. DE-OS 26 40 730). So können zum Beispiel 2-{4-[(6-Chlor-2-benzthiazolyl)-oxy]-phenoxy}-propionsäure-ethylester und 2-{4-[(6-Chlor-2-benzoxazolyl)-oxy]-phenoxy}-propionsäure-ethylester zur Bekämpfung von Unkraut eingesetzt werden. Die Wirkung dieser Stoffe ist gut, jedoch werden bei geringen Aufwandmengen einige Unkräuter nicht immer voll erfaßt. Außerdem läßt auch die Selektivität in manchen Fällen zu wünschen übrig.

Es wurden nun neue Phenoxypropionsäure-Derivate der Formel

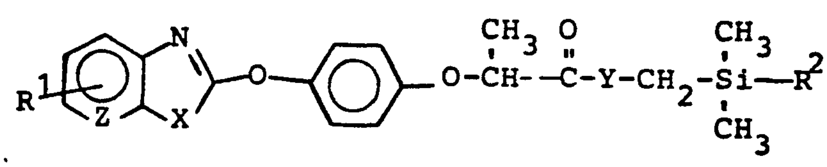

(I)

in welcher

X für Sauerstoff oder Schwefel steht,

Y für Sauerstoff, Schwefel oder die Gruppierung der Formel NR steht, wobei R für Wasserstoff oder Alkyl mit 1 bis 6 Kohlenstoffatomen steht,

Z für Stickstoff oder die CH-Gruppe steht,

$R^1$ für Wasserstoff oder Halogen steht und

$R^2$ für Alkyl mit 1 bis 6 Kohlenstoffatomen steht,

gefunden.

Die Phenoxypropionsäure-Derivate der Formel (I) enthalten mindestens ein asymmetrisch substituiertes Kohlenstoffatom und können deshalb in verschiedenen enantiomeren Formen vorliegen. Die Erfindung betrifft sowohl die möglichen einzelnen Isomeren als auch Gemische dieser Isomeren.

Weiterhin wurde gefunden, daß man Phenoxypropionsäure-Derivate der Formel (I) erhält, wenn man

a) Propionsäure-Derivate der Formel

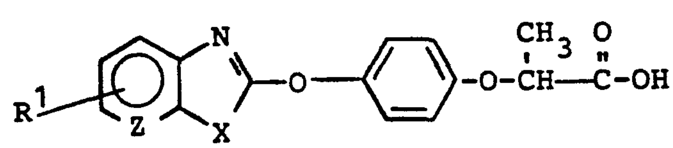

(II)

in welcher

$R^1$, X und Z die oben angegebene Bedeutung haben,

mit Silylchloriden der Formel

$$Cl-CH_2-\overset{\displaystyle CH_3}{\underset{\displaystyle CH_3}{\overset{|}{\underset{|}{Si}}}}-R^2 \qquad (III)$$

in welcher

$R^2$ die oben angegebene Bedeutung hat,

gegebenenfalls in Gegenwart eines Säurebindemittels sowie gegebenenfalls in Gegenwart eines Verdünnungsmittels umsetzt,

oder

b) Phenoxypropionsäurechloride der Formel

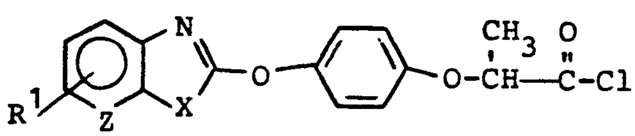

(IV)

in welcher

$R^1$, X und Z die oben angegebene Bedeutung haben,

mit Verbindungen der Formel

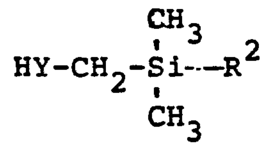

(V)

in welcher

$R^2$ und Y die oben angegebene Bedeutung haben,

gegebenenfalls in Gegenwart eines Säurebindemittels sowie gegebenenfalls in Gegenwart eines Verdünnungsmittels umsetzt, oder

c) Heterocyclen der Formel

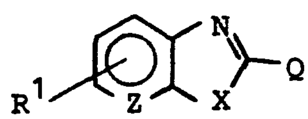

(VI)

in welcher
$R^1$, X und Z die oben angegebene Bedeutung haben und

Q für Halogen, Methylsulfonyl, Ethylsulfonyl oder Tosyl steht,

mit Hydrochinon-Derivaten der Formel

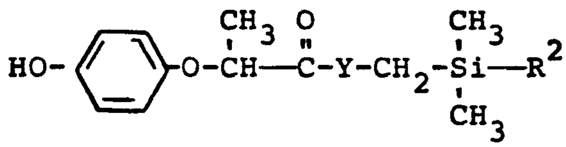

(VII)

in welcher

$R^2$ und Y die oben angegebene Bedeutung haben,

gegebenenfalls in Gegenwart eines Säurebindemittels sowie gegegebenenfalls in Gegenwart eines Verdünnungsmittels umsetzt.

Schließlich wurde gefunden, daß sich die neuen Phenoxypropionsäure-Derivate der Formel (I) durch eine hervorragende herbizide Wirksamkeit auszeichnen.

Überraschenderweise besitzen die erfindungsgemäßen Phenoxypropionsäure-Derivate der Formel (I) wesentlich bessere herbizide Eigenschaften als der 2-{4-[(6-Chlor-2-benzthiazolyl)-oxy]-phenoxy}-propionsäure-ethylester und der 2-{4-[(6-Chlor-2-benzoxazolyl)-oxy]-phenoxy}-propionsäure-ethylester, welches die konstitutionell ähnlichsten vorbekannten Wirkstoffe gleicher Wirkungsart sind.

Die erfindungsgemäßen Phenoxypropionsäure-Derivate sind durch die Formel (I) allgemein definiert. In dieser Formel steht

X für Sauerstoff oder Schwefel,

Y vorzugsweise für Sauerstoff, Schwefel oder die Gruppierung der Formel NR, worin

R vorzugsweise für Wasserstoff oder Alkyl mit 1 bis 4 Kohlenstoffatomen steht,

Z vorzugsweise für Stickstoff oder die CH-Gruppe,

$R^1$ vorzugsweise für Wasserstoff, Fluor, Chlor oder Brom und

$R^2$ vorzugsweise für Alkyl mit 1 bis 4 Kohlenstoffatomen.

Besonders bevorzugt sind diejenigen Verbindungen der Formel (I), in denen

X für Sauerstoff oder Schwefel steht,

Y für Sauerstoff, Schwefel oder die Gruppierung der Formel NR steht, worin

R für Wasserstoff, Methyl, Ethyl, n-Propyl oder Isopropyl steht,

Z für Stickstoff oder die CH-Gruppe steht,

$R^1$ für Wasserstoff, Fluor oder Chlor steht und

$R^2$ für Methyl, Ethyl, n-Propyl oder n-Butyl steht.

Eine weitere Gruppe besonders bevorzugter erfindungsgemäßer Verbindungen sind schließlich diejenigen Phenoxypropionsäure-Derivate der Formel (I), in denen das asymmetrisch substituierte Kohlenstoffatom der Propionsäure-Einheit die R-Konfiguration aufweist und die Reste X, Y, Z, $R^1$ und $R^2$ die oben angegebenen besonders bevorzugten Bedeutungen haben. Die betreffenden Stoffe lassen sich durch die Formel

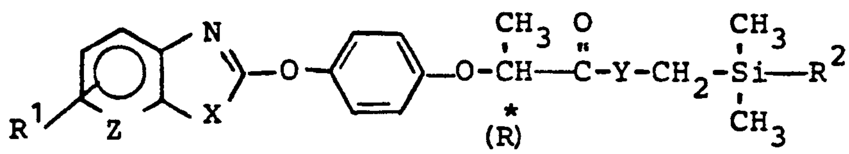

(Ia)

charakterisieren. In dieser Formel (Ia) ist das asymmetrisch substituierte Kohlenstoffatom durch ein (*) gekennzeichnet.

Als Beispiele für Phenoxypropionsäure-Derivate der Formel (I) seien die in der folgenden Tabelle formelmäßig aufgeführten Verbindungen genannt:

Tabelle 1

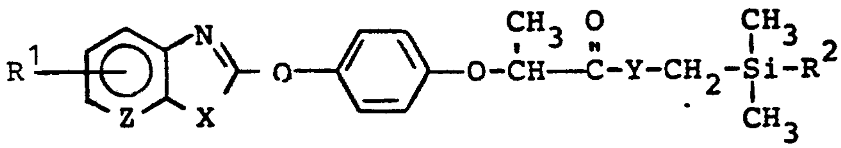

(I)

| $R^1$ | Z | X | Y | $R^2$ |
|---|---|---|---|---|
| H | CH | O | O | $CH_3$ |
| 6-Cl | CH | O | O | $CH_3$ |
| H | N | O | O | $CH_3$ |
| 6-Cl | N | O | O | $CH_3$ |
| H | CH | S | O | $CH_3$ |
| 6-Cl | CH | S | O | $CH_3$ |
| H | N | S | O | $CH_3$ |
| H | N | S | O | $C_2H_5$ |
| 6-Cl | N | S | O | $CH_3$ |
| H | CH | O | O | $C_2H_5$ |

0175199

Tabelle 1 (Fortsetzung)

| $R^1$ | Z | X | Y | $R^2$ |
|---|---|---|---|---|
| 6-Cl | CH | O | O | $C_2H_5$ |
| H | CH | O | O | $n-C_3H_7$ |
| 6-Cl | CH | O | O | $n-C_3H_7$ |
| H | CH | O | O | $n-C_4H_9$ |
| 6-Cl | CH | O | O | $n-C_4H_9$ |
| 6-Cl | CH | O | NH | $CH_3$ |
| 6-Cl | CH | O | NH | $C_2H_5$ |
| 6-Cl | CH | O | NH | $n-C_3H_7$ |
| 6-Cl | CH | O | NH | $n-C_4H_9$ |
| 6-Cl | CH | O | $NCH_3$ | $CH_3$ |
| 6-Cl | CH | O | $NCH_3$ | $C_2H_5$ |
| 6-Cl | CH | O | $NCH_3$ | $n-C_3H_7$ |
| 6-Cl | CH | O | $NCH_3$ | $n-C_4H_9$ |

Tabelle 1 (Fortsetzung)

| $R^1$ | Z | X | Y | $R^2$ |
|---|---|---|---|---|
| 6-Cl | CH | S | NH | $CH_3$ |
| 6-Cl | CH | S | NH | $C_2H_5$ |
| 6-Cl | CH | S | NH | $n-C_4H_9$ |
| 6-Cl | CH | S | $NCH_3$ | $CH_3$ |
| 6-Cl | CH | S | $NCH_3$ | $n-C_3H_7$ |
| 6-Cl | CH | S | S | $C_2H_5$ |
| 6-Cl | CH | S | S | $n-C_4H_9$ |
| 6-Cl | CH | O | S | $CH_3$ |
| 6-Cl | CH | O | S | $C_2H_5$ |
| 6-Cl | CH | O | S | $n-C_3H_7$ |
| 6-Cl | CH | O | S | $n-C_4H_9$ |

Verwendet man 2-{4-[(2-Benzthiazolyl)-oxy]-phenoxy}-propionsäure und Chlormethyl-trimethylsilan als Ausgangssubstanzen, so kann der Verlauf des erfindungsgemäßen

Verfahrens (a) durch das folgende Formelschema wiedergegeben werden:

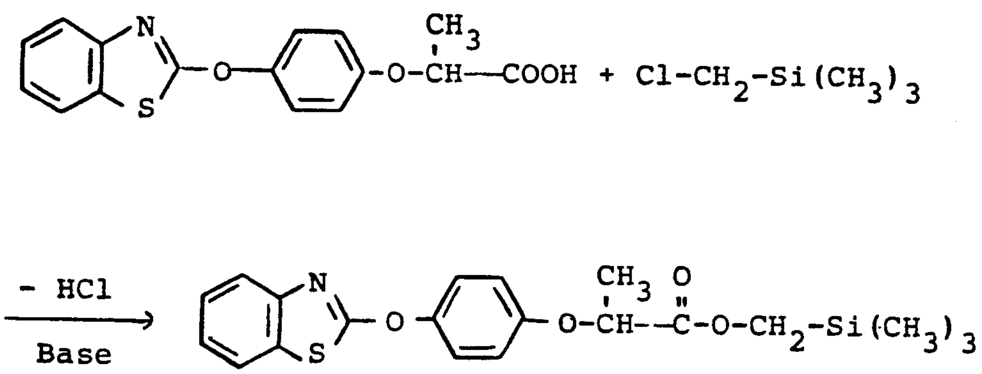


Verwendet man 2-{4-[(6-Chlor-2-benzoxazolyl)-oxy]-phenoxy}-propionsäurechlorid und (Trimethylsilylmethyl)-mercaptan als Ausgangsstoffe, so kann der Verlauf des erfindungsgemäßen Verfahrens (b) durch das folgende Formelschema wiedergegeben werden:

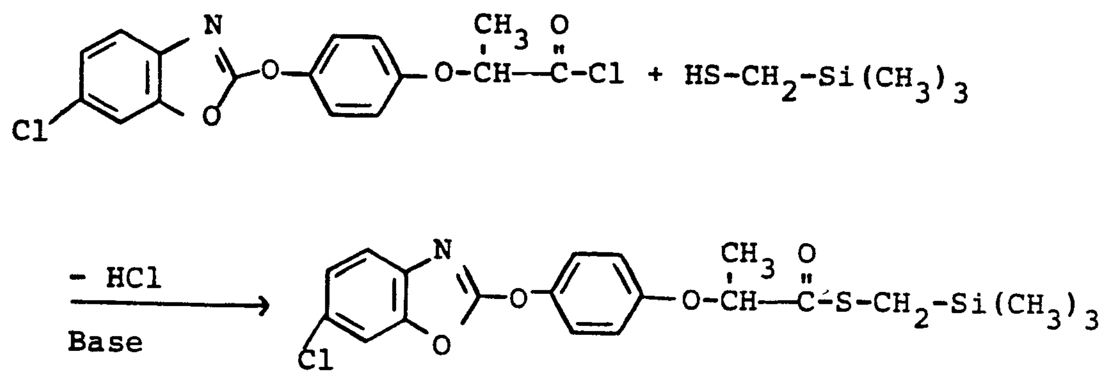


Verwendet man 2-Chlor-benzthiazol und 2-(4-Hydroxyphenoxy)-propionsäure-(trimethylsilyl-methyl)-ester als Ausgangssubstanzen, so kann der Verlauf des erfindungsgemäßen Verfahrens (c) durch das folgende Formelschema wiedergegeben werden:

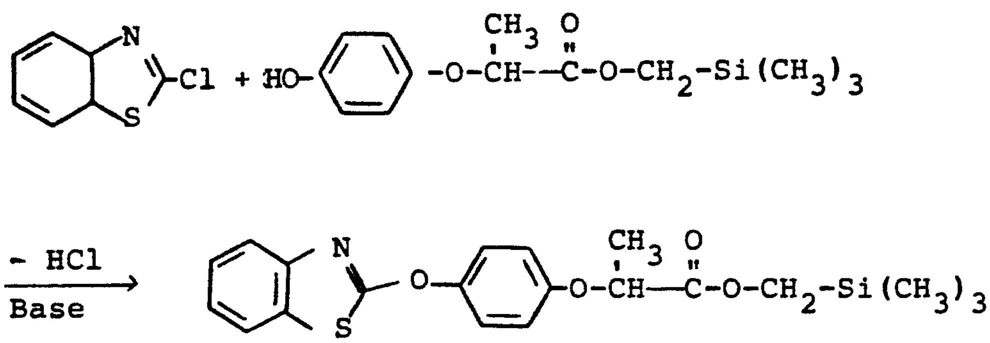


Die bei dem erfindungsgemäßen Verfahren (a) als Ausgangsstoffe benötigten Propionsäure-Derivate sind durch die Formel (II) definiert. In dieser Formel haben $R^1$, X und Z vorzugsweise diejenigen Bedeutungen, die bereits im Zusammenhang mit der Beschreibung der erfindungsgemäßen Stoffe der Formel (I) vorzugsweise für diese Reste genannt wurden.

Die Propionsäure-Derivate der Formel (II) sind bekannt oder lassen sich nach bekannten Verfahren in einfacher Weise herstellen (vgl. DE-OS 2 640 730, EP-OS 0 075 840 und EP-OS 0 002 800).

Zur Herstellung von R- und S-Enantiomeren der Phenoxypropionsäure-Derivate der Formel

(Ia) werden bei der Durchführung des erfindungsgemäßen Verfahrens (a) optisch aktive R- oder S-Propionsäure-Derivate der Formel (II) als Ausgangsstoffe benötigt. Auch diese optisch aktiven Propionsäure-Derivate der Formel (II) sind bekannt oder lassen sich nach prinzipiell bekannten Verfahren in einfacher Weise herstellen (vgl. EP-OS 0 002 800).

Man erhält R- bzw. S-Enantiomere der Propionsäure-Derivate der Formel

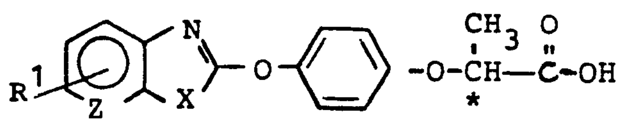

(II)

in welcher

$R^1$, X und Z die oben angegebene Bedeutung haben,

indem man Phenol-Derivate der Formel

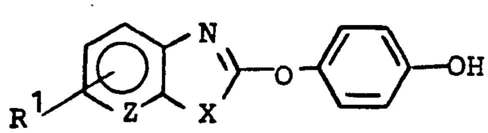

(VIII)

in welcher

$R^1$, X und Z die oben angegebene Bedeutung haben,

mit den S-Enantiomeren bzw. R-Enantiomeren von Propionsäure-Derivaten der Formel

$$Q^1-\underset{*}{\overset{CH_3}{C}}H-COOR^3 \qquad (IX)$$

in welcher

$Q^1$ für Chlor, Brom, Tosylat oder Mesylat steht und

$R^3$ für Methyl oder Ethyl steht,

gegebenenfalls in Gegenwart eines Säureakzeptors, wie z.B. Kaliumcarbonat, und gegebenenfalls in Gegenwart eines Verdünnungsmittels, wie zum Beispiel Acetonitril, bei Temperaturen zwischen 0°C und 120°C umsetzt und die dabei entstehenden Ester der Formel

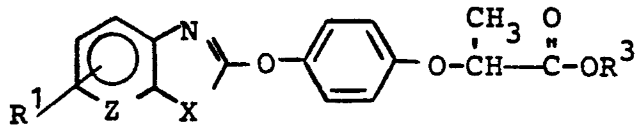

(X)

in welcher

$R^1$, $R^3$, X und Z die oben angegebene Bedeutung haben,

mit starken Basen, wie zum Beispiel Natriumhydroxid, in Gegenwart eines Verdünnungsmittels, wie zum Beispiel Methanol, Ethanol, Benzol, Toluol, Xylol, gegebenenfalls im Gemisch mit Wasser bei Temperaturen zwischen 20°C und 140°C verseift und anschließend mit einer Säure, wie zum Beispiel Salzsäure, ansäuert.

In der ersten Stufe dieser Umsetzung findet am asymmetrischen Kohlenstoffatom der Propionsäure-Einheit eine Walden'sche Umkehr statt. Dieses hat zur Folge, daß durch Umsetzung von Phenol-Derivaten der Formel (VIII) mit S-Enantiomeren der Propionsäure-Derivate der Formel (IX) die R-Enantiomeren der Propionsäure-Derivate der Formel (X) entstehen. Andererseits bilden sich durch Umsetzung der R-Enatiomeren von Phenol-Derivaten der Formel (VIII) mit R-Enantiomeren der Propionsäure-Derivate der Formel (IX) die S-Enantiomeren der Propionsäure-Derivate der Formel (X).

Die bei dem erfindungsgemäßen Verfahren (a) weiterhin als Ausgangsstoffe benötigten Silylchloride sind durch die Formel (III) definiert. In dieser Formel hat $R^2$ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Silylchloride der Formel (III) sind bekannt oder lassen sich in einfacher Weise nach bekannten Verfahren herstellen.

Die bei dem erfindungsgemäßen Verfahren (b) als Ausgangsstoffe benötigten Phenoxypropionsäurechloride sind durch

die Formel (IV) definiert. In dieser Formel haben $R^1$, X und Z vorzugsweise diejenigen Bedeutungen, die bereits im Zusammenhang mit der Beschreibung der erfindungsgemäßen Stoffe der Formel (I) vorzugsweise für diese Reste genannt wurden.

Die Phenoxypropionsäurechloride der Formel (IV) sind bekannt oder lassen sich nach bekannten Verfahren in einfacher Weise herstellen (vgl. DE-OS 2 640 730, EP-OS 0 075 840 und EP-OS 0 002 800). So lassen sich die Phenoxypropionsäurechloride der Formel (IV) aus den entsprechenden Propionsäure-Derivaten der Formel (II) durch Chlorierung, zum Beispiel mit Thionylchlorid, nach üblichen Methoden herstellen.

Die bei dem erfindungsgemäßen Verfahren (b) weiterhin als Ausgangsstoffe benötigten Verbindungen sind durch die Formel (V) definiert. In dieser Formel haben $R^2$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Verbindungen der Formel (V) sind bekannt oder lassen sich nach bekannten Methoden in einfacher Weise herstellen.

Zur Herstellung von R- bzw. S-Enantiomeren der Phenoxypropionsäure-Derivate der Formel (I) werden bei der Durchführung des erfindungsgemäßen Verfahrens (b) die R- bzw. S-Enantiomeren der Phenoxypropionsäurechloride der Formel (IV) eingesetzt.

Die bei dem erfindungsgemäßen Verfahren (c) als Ausgangsstoffe benötigten Heterocyclen sind durch die Formel (VI) definiert. In dieser Formel haben $R^1$, Z und X vorzugsweise diejenigen Bedeutungen, die bereits im Zusammenhang mit der Beschreibung der erfindungsgemäßen Stoffe der Formel (I) vorzugsweise für diese Reste genannt wurden. Q steht vorzugsweise für Fluor, Chlor, Brom, Methylsulfonyl, Ethylsulfonyl oder Tosyl.

Die Heterocyclen der Formel (VI) sind bekannt (vgl. DE-OS 2 640 730, EP-OS 0 044 497 und EP-OS 0 075 840).

Die bei dem erfindungsgemäßen Verfahren (c) weiterhin als Ausgangsstoffe benötigten Hydrochinon-Derivate sind durch die Formel (VII) definiert. In dieser Formel haben $R^2$ und Y vorzugsweise diejenigen Bedeutungen, die bereits im Zusammenhang mit der Beschreibung der erfindungsgemäßen Stoffe der Formel (I) vorzugsweise für diese Reste genannt wurden.

Die Hydrochinon-Derivate der Formel (VII) sind teilweise bekannt (vgl. EP-OS 0 095 115). Sie lassen sich herstellen, indem man in einer ersten Stufe Hydrochinonmonobenzylether der Formel

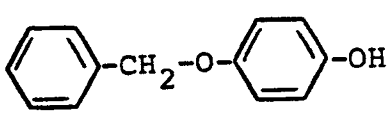

(XI)

mit Silylverbindungen der Formel

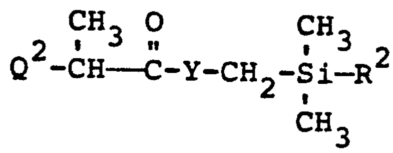

(XII)

in welcher

$R^2$ und Y die oben angegebene Bedeutung haben und

$Q^2$ für Chlor, Brom, Tosylat oder Mesylat steht,

gegebenenfalls in Gegenwart eines Säurebindemittels sowie gegebenenfalls in Gegenwart eines polaren Verdünnungsmittels, wie zum Beispiel Dimethylformamid, Dimethylsulfoxid, Acetonitril oder Methylisobutylketon, bei Temperaturen zwischen 40 und 120°C, vorzugsweise zwischen 60 und 100°C, umsetzt und in einer zweiten Stufe die entstehenden Hydrochinonether der Formel

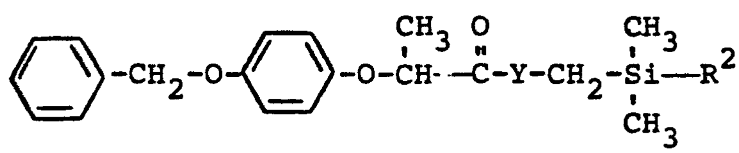

(XIII)

in welcher

$R^2$ und Y die oben angegebene Bedeutung haben,

mit Wasserstoff unter einem Druck von 10 bar bis 100 bar in Gegenwart eines Katalysators, wie zum Beispiel Palladium auf Aktivkohle, und in Gegenwart eines Verdünnungsmittels, wie zum Beispiel Essigester, bei Temperaturen zwischen 20 und 60°C hydriert.

Als Säureakzeptoren können in der ersten Stufe dieser Umsetzung vorzugsweise alle diejenigen Säurebindemittel eingesetzt werden, die im Zusammenhang mit der Beschreibung der erfindungsgemäßen Verfahren (a) bis (c) vorzugsweise als Säureakzeptoren genannt werden (vgl. unten).

Der bei dem obigen Verfahren zur Herstellung von Hydrochinon-Derivaten der Formel (VII) als Ausgangsstoff benötigte Hydrochinon-monobenzylether der Formel (XI) ist bekannt (vgl. J. Org. Chem. 39, (1974) 214-215).

Die bei dem obigen Verfahren zur Herstellung von Hydrochinon-Derivaten der Formel (VII) weiterhin als Ausgangsstoffe benötigten Silylverbindungen der Formel (XII) sind teilweise bekannt (vgl. EP-OS 0 095 115 und EP-OS 0 096 354). Sie lassen sich herstellen, indem man Propionsäure-Derivate der Formel

$$Q^3-\overset{CH_3}{\overset{|}{C}H}-\overset{O}{\overset{\|}{C}}-Cl \qquad (XIV)$$

in welcher

$Q^3$ für Chlor, Brom, Mesylat oder Tosylat steht,

mit Verbindungen der Formel

$$HY\text{-}CH_2\text{-}\overset{\displaystyle CH_3}{\underset{\displaystyle CH_3}{Si}}\text{—}R^2 \qquad (V)$$

in welcher

$R^2$ und Y die oben angegebene Bedeutung haben,

gegebenenfalls in Gegenwart eines Säurebindemittels und gegebenenfalls in Gegenwart eines Verdünnungsmittels umsetzt. Die Umsetzungsbedingungen bei diesem Verfahren entsprechen denjenigen des erfindungsgemäßen Verfahrens (b) (vgl. unten).

Die bei dem Verfahren zur Herstellung von Hydrochinon-Derivaten der Formel (VII) als Zwischenprodukte anfallenden Hydrochinonether der Formel (XIII) sind neu.

Zur Herstellung von R- bzw. S-Enantiomeren der Phenoxypropionsäure-Derivate der Formel (I) werden bei der Durchführung des erfindungsgemäßen Verfahrens (c) die R- bzw. S-Enantiomeren der Hydrochinon-Derivate der Formel (VII) eingesetzt. Auch diese optisch aktiven Stoffe lassen sich nach dem oben beschriebenen Verfahren zur Herstellung von Hydrochinon-Derivaten der Formel (VII) synthetisieren, indem man entsprechende optisch aktive Silylverbindungen der Formel (XII) einsetzt.

Die erfindungsgemäßen Verfahren (a), (b) und (c) zur Herstellung der neuen Phenoxypropionsäure-Derivate der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutyl-keton, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon sowie Dimethylsulfoxid, Tetramethylensulfon und Hexamethylphosphorsäuretriamid.

Als Säurebindemittel können sowohl bei den erfindungsgemäßen Verfahren (a) und (b) als auch bei dem Verfahren (c) alle üblicherweise für derartige Umsetzungen verwendbaren Säurebindemittel eingesetzt werden. Vorzugsweise in Frage kommenden Alkalihydroxide, wie z.B. Natrium- und Kaliumhydroxid, Erdalkalihydroxyde, z. B. Calziumhydroxyd, Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diaza-bicyclo-[4.3.0]-nonen-5(DBN) und 1,8-Diazabicyclo-[5.4.0]-undec-7-en (DBU).

Die Reaktionstemperaturen können sowohl bei dem erfindungsgemäßen Verfahren (a) als auch bei den Verfahren (b) und (c) innerhalb eines größeren Bereiches variiert werden. Im allgemeinen arbeitet man jeweils bei Temperaturen zwischen -20°C und +160°C, vorzugsweise zwischen 0°C und 140°C.

Die erfindungsgemäßen Verfahren (a), (b) und (c) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a), (b) und (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a), (b) und (c) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob

0175199

die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich sehr gut zur selektiven Unkrautbekämpfung in Getreide und Reis. Sie besitzen vor allem eine sehr gute Wirkung gegen grasartige Unkräuter.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in

Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phosphorlipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z.B. N-(2-Benzthiazolyl)-N,N'-dimethyl-harnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5(4H)-on, 4-Amino-6-(1,1-dimethyl-ethyl)-3-ethylthio-1,2,4-triazin-5(4H)-on, 1-Amino-6-ethylthio-3-(2,2-dimethylpropyl)-1,3,5-triazin-2,4-(1H, 3H)-dion, 4-Amino-3-methyl-6-phenyl-1,2,4-triazin-5(4H)-on; 2-Chlor-4-ethylamino-6-isopropyl-amino-1,3,5-triazin, das R-Enantiomere des 2-/4-(3,5-Dichlor-pyridin-2-oxy)-phenoxy7-propionsäure-(trimethylsilyl)-methylesters, das R-Enantiomere des 2-/4-(3,5-Dichlorpyridyl-2-oxy)-phenoxy7-propionsäure-(2-benzyloxy)-ethylesters, 2,4-Dichlorphenoxyessigsäure, 2-(2,4-Dichlorphenoxy)-propionsäure, 4-Chlor-2-methyl-phenoxy-essigsäure, 2-(2-Methyl-3-chlor-phenoxy)-propionsäure, 3,5-Dijod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor

als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

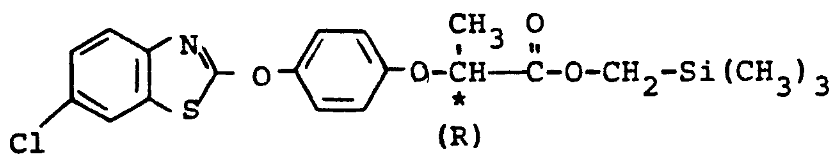

(I-1)

Eine Lösung von 6,2 g (0,179 Mol) des R-Enantiomeren der 2-{4-[(6-Chlor-2-benzthiazolyl)-oxy]-phenoxy}-propionsäure in 20 ml Dimethylformamid wird mit 2,9 g Kaliumcarbonat versetzt. Nach 15-minütigem Rühren bei 50°C werden bei dieser Temperatur 2,7 g (0,021 Mol) Chlormethyltrimethylsilan zugetropft. Anschließend wird 2 Stunden bei 80°C nachgerührt. Danach wird aufgearbeitet, indem man das Lösungsmittel unter vermindertem Druck abzieht, den Rückstand mit Wasser versetzt und das anfallende ölige Produkt mit 100 ml Toluol extrahiert. Die organische Phase wird nacheinander mit wäßriger Natriumcarbonat-Lösung und mit Wasser gewaschen, getrocknet und unter vermindertem Druck eingeengt. Man erhält auf diese Weise 7 g (89 % der Theorie) an R-Enantiomerem des 2-{4-[(6-Chlor-2-benzthiazolyl)-oxy]-phenoxy}-propionsäure-(trimethylsilyl-methyl)-esters in Form eines viskosen Öles.

Brechnungsindex: $n_D^{20}$ = 1,5771

Drehwert: $[\alpha]_D^{24}$ = + 12,20°

(1-molare Lösung in Chloroform; Küvettenlänge 10 cm)

Beispiel 2

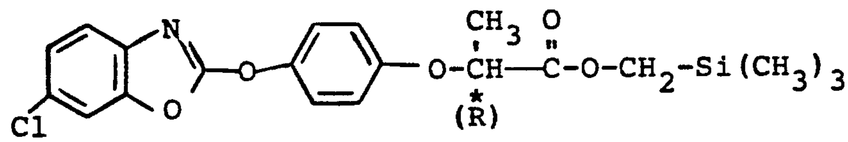

(I-2)

Ein Gemisch aus 6,7 g (0,025 Mol) des R-Enantiomeren des 2-(4-Hydroxy-phenoxy)-propionsäure-(trimethylsilyl-methyl)-esters, 0,9 g Calciumhydroxid und 10 ml Dimethylsulfoxid wird auf 40°C erhitzt. Bei dieser Temperatur wird eine Lösung von 4,7 g (0,025 Mol) 2,6-Dichlorbenzoxazol in 15 ml Dimethylsulfoxid unter Rühren langsam zugetropft. Das Reaktionsgemisch wird 2 Stunden bei 40°C und 16 Stunden bei Raumtemperatur nachgerührt. Danach wird aufgearbeitet, indem man das Reaktionsgemisch in Wasser gießt, das anfallende ölige Produkt mit Cyclohexan extrahiert, die organische Phase nacheinander mit verdünnter wäßriger Natronlauge und Wasser wäscht, mit Eisessig schwach sauer stellt, dann trocknet und unter vermindertem Druck einengt. Man erhält auf diese Weise 9,3 g (88,6 % der Theorie) an dem R-Enantiomeren des 2-{4-[(6-Chlor-2-benzoxazolyl)-oxy]-phenoxy}-propionsäure-(trimethylsilyl-methyl)-esters in kristalliner Form.

Schmelzpunkt: 68°C.

Drehwert: $[\alpha]_D^{24}$ = + 10,3°

(1-molare Lösung in Chloroform; Küvettenlänge 10 cm)

## Beispiel 3

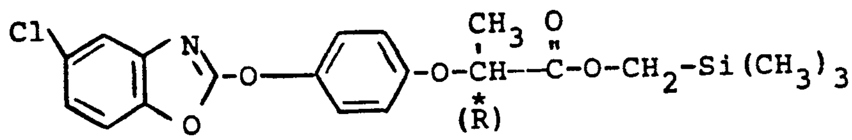 (I-3)

Nach der im Beispiel 2 angegebenen Methode wird auch die Verbindung der Formel (I-3) hergestellt.

Brechungsindex: $n_D^{20}$ = 1,5204
Drehwert: $[\alpha]_D^{24}$ = + 11,6°
(1-molare Lösung in Chloroform; Küvettenlänge 10 cm)

## Beispiel 4

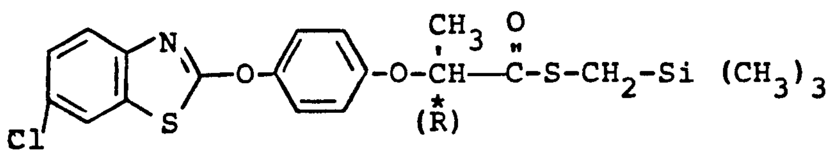 (I-4)

In ein Gemisch aus 1,56 g (0,013 Mol) (Trimethylsilylmethyl)-mercaptan, 1,6 g (0,016 Mol) Triethylamin und 20 ml Toluol wird bei 0°C unter Rühren eine Lösung von 4,9 g (0,013 Mol) des R-Enantiomeren des 2- {4-[(6-Chlor-2-benzthiazolyl)-oxy]-phenoxy} -propionsäurechlorids in 10 ml Toluol eingetropft. Es wird 3 Stunden bei Temperaturen zwischen 0°C und 5°C nachgerührt und dann aufgearbeitet, indem man das Reaktionsgemisch mit Wasser verdünnt, das anfallende ölige Produkt mit Toluol

extrahiert, die organische Phase schnell mit 5 %iger wäßriger Natronlauge wäscht, dann mit verdünnter Essigsäure und Wasser wäscht und nach dem Trocknen durch Abziehen des Verdünnungsmittels unter vermindertem Druck einengt. Man erhält auf diese Weise 4,5 g (77 % der Theorie) an dem R-Enantiomeren des 2-{4-[(6-Chlor-2-benzthiazolyl)-oxy]-phenoxy}-propionsäure-(trimethylsilyl-methyl)-thioesters. Schmelzpunkt: 90°C.

$[\alpha]_D^{24}$ = + 15,4° (1-molare Lösung in Chloroform; Kuvettenlänge 10 cm.)

In analoger Weise werden die in den folgenden Beispielen aufgeführten Stoffe erhalten:

__Beispiel 5__

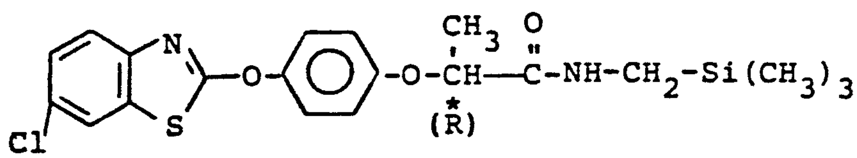
(I-5)

Schmelzpunkt: 88°C
Drehwert $[\alpha]_D^{24}$ = + 6,7° (1-molare Lösung in Chloroform; Küvettenlänge 10 cm).

__Beispiel 6__

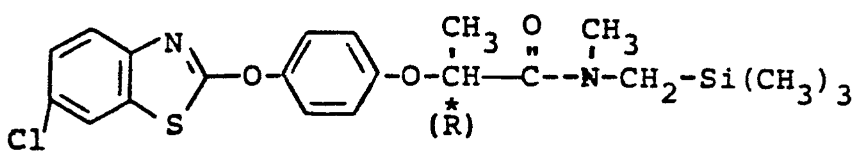
(I-6)

0175199

Schmelzpunkt: 70°C

Drehwert $[\alpha]_D^{24}$ = + 7,3° (1-molare Lösung in Chloroform; Küvettenlänge 10 cm)

Beispiel 7

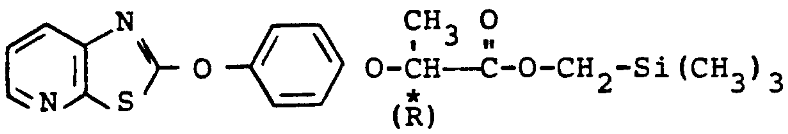

(I-7)

In ein Gemisch aus 13,4 g (0,05 Mol) R-Enantiomerem des 2-(4-Hydroxy-phenoxy)-propionsäure-(trimethylsilylmethyl)-esters, 1,85 g (0,025 Mol) Calciumhydroxid und 20 ml Dimethylsulfoxid wird bei 40°C unter Rühren eine Lösung von 10,7 g (0,05 Mol) 2-Methylsulfonyl-thiazolo-[5,4-b]pyridin in 30 ml Dimethylsulfoxid langsam eingetropft. Es wird 2 Stunden bei 40°C und 16 Stunden bei Raumtemperatur nachgerührt. Danach wird aufgearbeitet, indem man das Reaktionsgemisch in Wasser gießt, das anfallende ölige Produkt mit Chloroform extrahiert, die organische Phase schnell je einmal mit 5 %iger wäßriger Natronlauge, verdünnter Essigsäure und Wasser wäscht und nach dem Trocknen durch Abziehen des Verdünnungsmittels unter vermindertem Druck einengt. Man erhält auf diese Weise 19 g (94,5 % der Theorie) an dem R-Enantiomeren der Verbindung der oben angegebenen Formel (I-7).

Berechnungsindex: $n_D^{25}$ = 1,5530

0175199

Herstellung von Ausgangssubstanzen

Beispiel (VII-1)

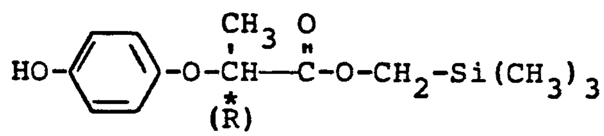

(VII-1)

206 g (0,57 Mol) an R-Enantiomerem des 2-/4-(Benzyloxy-phenoxy)7-propionsäure-(trimethylsilyl-methyl)-esters werden in 1200 ml Essigester gelöst und in Gegenwart von 40 g Palladium auf Aktivkohle (5 %ig) bei Temperaturen von 40 bis 60°C mit Wasserstoff unter einem Druck von 60 bar hydriert. Anschließend arbeitet man auf, indem man den Katalysator abfiltriert und durch Abziehen des Verdünnungsmittels unter vermindertem Druck einengt. Man erhält auf diese Weise 150 g (98 % der Theorie) an R-Enantiomerem des 2-(4-Hydroxy-phenoxy)-propionsäure-(trimethylsilyl-methyl)-esters.

Brechungsindex: $n_D^{24}$ = 1,4983

Drehwert: $[\alpha]_D^{24}$ = +6,64°
(1-molare Lösung in Chloroform;
Küvettenlänge 10 cm)

Nach der im Beispiel (VII-1) beschriebenen Methode lassen sich auch die in der folgenden Tabelle 2 aufgeführten Stoffe herstellen:

Tabelle 2

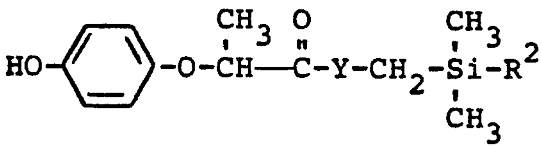

(VII)

| Beispiel Nr. | Y | $R^2$ |
|---|---|---|
| VII-2 | O | $C_2H_5$ |
| VII-3 | O | $n\text{-}C_3H_7$ |
| VII-4 | O | $n\text{-}C_4H_9$ |
| VII-5 | NH | $CH_3$ |
| VII-6 | NH | $C_2H_5$ |
| VII-7 | NH | $n\text{-}C_3H_7$ |
| VII-8 | NH | $n\text{-}C_4H_9$ |
| VII-9 | $NCH_3$ | $CH_3$ |
| VII-10 | $NCH_3$ | $C_2H_5$ |
| VII-11 | $NCH_3$ | $n\text{-}C_3H_7$ |
| VII-12 | $NCH_3$ | $n\text{-}C_4H_9$ |
| VII-13 | S | $CH_3$ |
| VII-14 | S | $C_2H_5$ |
| VII-15 | S | $n\text{-}C_3H_7$ |
| VII-16 | S | $n\text{-}C_4H_9$ |

Beispiel (VIII-1)

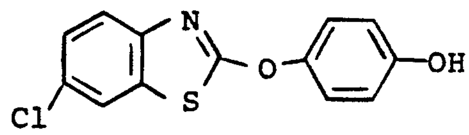

(VIII-1)

Ein Gemisch aus 11 g (0,1 Mol) Hydrochinon, 50 ml Dimethylsulfoxid und 3,7 g (0,05 Mol) Calciumhydroxid wird unter Stickstoff auf 80°C erhitzt. Danach wird unter Rühren bei dieser Temperatur eine Lösung von 10,2 g (0,05 Mol) 2,6-Dichlorbenzthiazol in 10 ml Dimethylsulfoxid langsam zugetropft. Es wird 8 Stunden bei 80°C nachgerührt. Anschließend wird aufgearbeitet, indem man das Reaktionsgemisch durch Abziehen des Lösungsmittels einengt, den verbleibenden Rückstand mit Wasser und wäßriger Salzsäure versetzt und das ausfallende Festprodukt absaugt und trocknet. Man erhält auf diese Weise 11,1 g (80 % der Theorie) an 4-(6-Chlor-2-benzthiazolyl)-oxyphenol.

Schmelzpunkt: 174°C (nach Umkristallisation aus Xylol).

Beispiel (VIII-2)

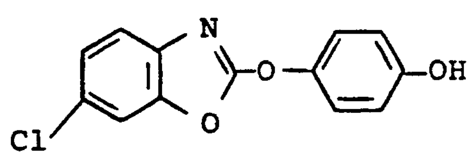

(VIII-2)

Nach der im Beispiel (VIII-1) beschriebenen Methode wird auch die Verbindung der Formel (VIII-2) hergestellt.

Ausbeute: 85 % der Theorie
Schmelzpunkt: 176°C (nach Umkristallisation aus Butanol)

Beispiel (XII-1)

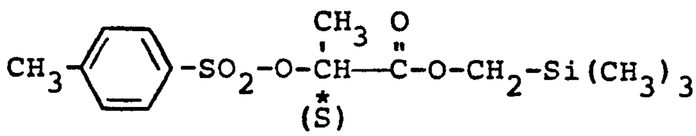 (XII-1)

In ein Gemisch aus 109 g (0,41 Mol) S-Enantiomerem des Tosyloxy-propionsäure-chlorids, 43,4 g (0.41 Mol) Trimethylsilyl-methanol und 250 ml Toluol werden bei 20°C unter Rühren langsam 33 g (0,41 Mol) Pyridin eingetropft. Es wird 24 Stunden bei Raumtemperatur nachgerührt. Danach wird aufgearbeitet, indem man das Reaktionsgemisch in Wasser gießt, mit Toluol extrahiert und die organische Phase durch Abziehen des Verdünnungsmittels unter vermindertem Druck einengt. Man erhält auf diese Weise 130 g (96 % der Theorie) an S-Enantiomerem des 2-Tosyloxy-propionsäure-(trimethylsilyl-methyl)-esters in Form eines öligen Produktes.
Siedepunkt: 145-155°C / 0,05 mbar.

Nach der im Beispiel (XII-1) beschriebenen Methode lassen sich auch die in der folgenden Tabelle 3 aufgeführten Stoffe herstellen:

Tabelle 3

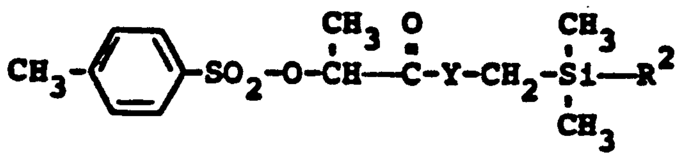

(XIIa)

| Beispiel Nr. | Y | $R^2$ |
|---|---|---|
| XII-2 | O | $C_2H_5$ |
| XII-3 | O | $n-C_3H_7$ |
| XII-4 | O | $n-C_4H_9$ |
| XII-5 | NH | $CH_3$ |
| XII-6 | NH | $C_2H_5$ |
| XII-7 | NH | $n-C_3H_7$ |
| XII-8 | NH | $n-C_4H_9$ |
| XII-9 | $NCH_3$ | $CH_3$ |
| XII-10 | $NCH_3$ | $C_2H_5$ |
| XII-11 | $NCH_3$ | $n-C_3H_7$ |
| XII-12 | $NCH_3$ | $n-C_4H_9$ |
| XII-13 | S | $CH_3$ |
| XII-14 | S | $C_2H_5$ |
| XII-15 | S | $n-C_3H_7$ |
| XII-16 | S | $n-C_4H_9$ |

Beispiel (XIII-1)

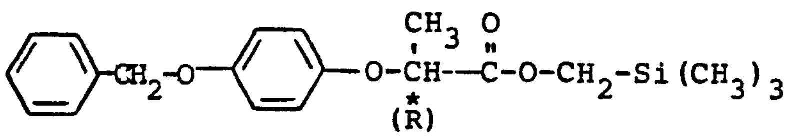

(XIII-1)

Ein Gemisch aus 100 g (0,5 Mol) Hydrochinon-monobenzylether, 165 g (0,5 Mol) an S-Enantiomerem des 2-Tosyloxy-propionsäure-(trimethylsilyl-methyl)-esters, 1000 ml Methyl-iso-butyl-keton und 138 g (1 Mol) Kaliumcarbonat wird 5 Stunden bei 85-90°C gerührt. Anschließend wird aufgearbeitet, indem man den festen Rückstand absaugt, das Lösungsmittel abdestilliert, den verbleibenden Rückstand in 800 ml Toluol löst, die organische Phase zweimal mit je 1000 ml 5 %iger wäßriger Natronlauge bei 40°C wäscht, dann die organische Phase mit wenig Eisessig sauer stellt und mit Wasser neutral wäscht. Nach dem Abziehen des Lösungsmittels unter vermindertem Druck erhält man 107 g (60 % der Theorie) an R-Enantiomerem des 2-/4-(Benzyloxy-phenoxy)7-propionsäure-(trimethylsilyl-methyl)-esters in Form eines Öles.

Brechungsindex: $n_D^{24}$ = 1,5216

Drehwert: $[\alpha]_D^{24}$ = + 7,31°

(1-molare Lösung in Chloroform, Küvettenlänge 10 cm)

Nach der im Beispiel (XIII-1) beschriebenen Methode lassen sich auch die in der folgenden Tabelle 4 aufgeführten Stoffe herstellen:

Tabelle 4

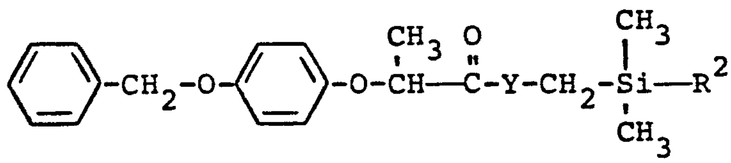

(XIII)

| Beispiel Nr. | Y | $R^2$ |
|---|---|---|
| XIII-2 | O | $C_2H_5$ |
| XIII-3 | O | $n-C_3H_7$ |
| XIII-4 | O | $n-C_4H_9$ |
| XIII-5 | NH | $CH_3$ |
| XIII-6 | NH | $C_2H_5$ |
| XIII-7 | NH | $n-C_3H_7$ |
| XIII-8 | NH | $n-C_4H_9$ |
| XIII-9 | $NCH_3$ | $CH_3$ |
| XIII-10 | $NCH_3$ | $C_2H_5$ |
| XIII-11 | $NCH_3$ | $n-C_3H_7$ |
| XIII-12 | $NCH_3$ | $n-C_4H_9$ |
| XIII-13 | S | $CH_3$ |
| XIII-14 | S | $C_2H_5$ |
| XIII-15 | S | $n-C_3H_7$ |
| XIII-16 | S | $n-C_4H_9$ |

In den nachstehend beschriebenen biologischen Tests wurde die folgende Verbindung als Vergleichssubstanz eingesetzt:

(A) = 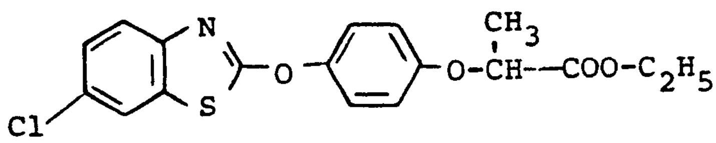

2-{4-[(6-Chlor-2-benzthiazolyl)-oxy]-phenoxy}-propionsäure-ethylester

(bekannt aus DE-OS 26 40 730)

Beispiel A

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt der Wirkstoff gemäß Beispiel 1 bei der Bekämpfung von Setaria in Weizen bei einer Aufwandmenge von 0,5 kg/ha eine bessere selektive herbizide Wirksamkeit als die Vergleichssubstanz (A).

Beispiel B

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigt der Wirkstoff gemäß Beispiel 1 bei der Bekämpfung von Digitaria und Agropyron in Zuckerrüben eine bessere selektive herbizide Wirksamkeit als die Vergleichssubstanz (A), die Wirkstoffe gemäß den Beispielen 2 und 3 zeigen im Weizen eine bessere selektive herbizide Wirksamkeit als die Vergleichssubstanz (A).

Patentansprüche

1. Phenoxypropionsäure-Derivate der Formel

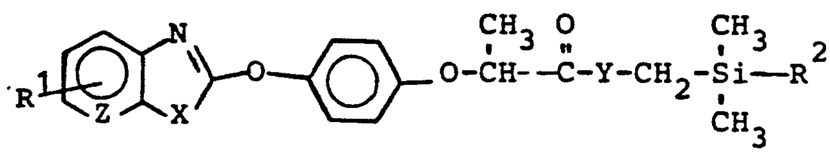

(I)

in welcher

X für Sauerstoff oder Schwefel steht,

Y für Sauerstoff, Schwefel oder die Gruppierung der Formel NR steht, wobei R für Wasserstoff oder Alkyl mit 1 bis 6 Kohlenstoffatomen steht,

Z für Stickstoff oder die CH-Gruppe steht,

$R^1$ für Wasserstoff oder Halogen steht und

$R^2$ für Alkyl mit 1 bis 6 Kohlenstoffatomen steht.

2. Phenoxypropionsäure-Derivate der Formel (I) gemäß Anspruch 1, in denen

X für Sauerstoff oder Schwefel steht,

Y für Sauerstoff, Schwefel oder die Gruppierung der Formel NR steht, worin
R für Wasserstoff oder Alkyl mit 1 bis 4 Kohlenstoffatomen steht,

Z für Stickstoff oder die CH-Gruppe steht,

$R^1$ für Wasserstoff, Fluor, Chlor oder Brom steht und

$R^2$ für Alkyl mit 1 bis 4 Kohlenstoffatomen steht.

3. R-Enantiomere von Phenoxypropionsäure-Derivaten der Formel

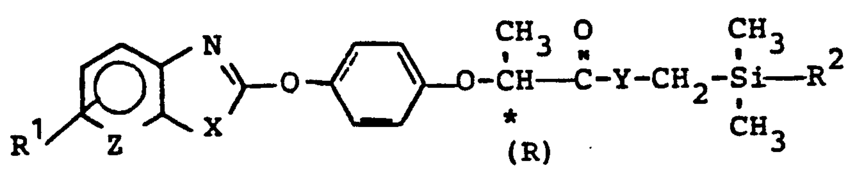

(Ia)

in welcher

X für Sauerstoff oder Schwefel steht,

Y für Sauerstoff, Schwefel oder die Gruppierung der Formel NR steht, worin
R für Wasserstoff oder Alkyl mit 1 bis 4 Kohlenstoffatomen steht,

Z für Stickstoff oder die CH-Gruppe steht,

$R^1$ für Wasserstoff, Fluor, Chlor oder Brom steht und

$R^2$ für Alkyl mit 1 bis 4 Kohlenstoffatomen steht.

4. Verfahren zur Herstellung von Phenoxypropionsäure-Derivate der Formel

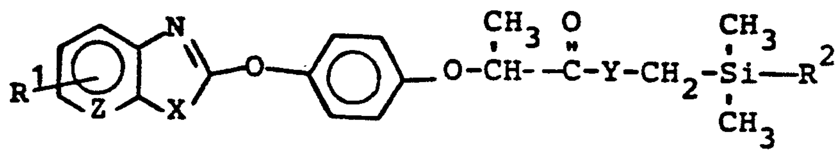

(I)

in welcher

X für Sauerstoff oder Schwefel steht,

Y für Sauerstoff, Schwefel oder die Gruppierung der Formel NR steht, wobei
R für Wasserstoff oder Alkyl mit 1 bis 6 Kohlenstoffatomen steht,

Z für Stickstoff oder die CH-Gruppe steht,

$R^1$ für Wasserstoff oder Halogen steht und

$R^2$ für Alkyl mit 1 bis 6 Kohlenstoffatomen steht,

dadurch gekennzeichnet, daß man

a) Propionsäure-Derivate der Formel

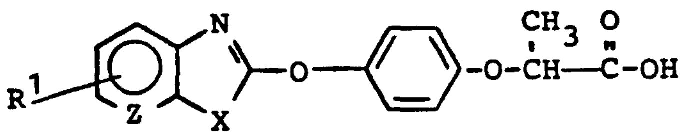

(II)

in welcher

$R^1$, X und Z die oben angegebene Bedeutung haben,

mit Silylchloriden der Formel

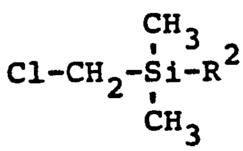

(III)

in welcher

$R^2$ die oben angegebene Bedeutung hat,

gegebenenfalls in Gegenwart eines Säurebindemittels sowie gegebenenfalls in Gegenwart eines Verdünnungsmittels umsetzt,

oder

b) Phenoxypropionsäurechloride der Formel

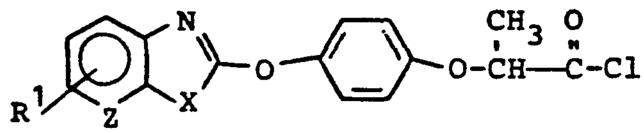

(IV)

in welcher

$R^1$, X und Z die oben angegebene Bedeutung haben,

mit Verbindungen der Formel

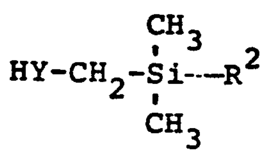

(V)

in welcher

$R^2$ und Y die oben angegebene Bedeutung haben,

gegebenenfalls in Gegenwart eines Säurebindemittels sowie gegebenenfalls in Gegenwart eines Verdünnungsmittels umsetzt,

oder

c) Heterocyclen der Formel

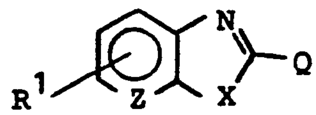

(VI)

in welcher

$R^1$, X und Z die oben angegebene Bedeutung haben und

Q für Halogen, Methylsulfonyl, Ethylsulfonyl oder Tosyl steht,

mit Hydrochinon-Derivaten der Formel

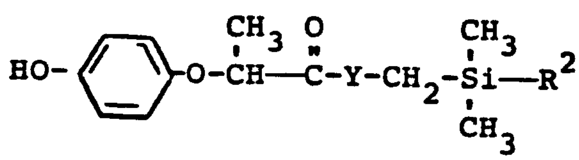

(VII)

in welcher

$R^2$ und Y die oben angegebene Bedeutung haben,

gegebenenfalls in Gegenwart eines Säurebindemittels sowie gegebenenfalls in Gegenwart eines Verdünnungsmittels umsetzt.

5. Herbizide Mittel, gekennzeichnet durch einen Gehalt an mindestens einem Phenoxypropionsäure-Derivat der Formel (I) gemäß den Ansprüchen 1 bis 4.

6. Verfahren zur Bekämpfung von Unkräutern, dadurch gekennzeichnet, daß man Phenoxypropionsäure-Derivate der Formel (I) gemäß den Ansprüchen 1 bis 4 auf die Unkräuter und/oder deren Lebensraum ausbringt.

7. Verwendung von Phenoxypropionsäure-Derivaten der Formel (I) gemäß den Ansprüchen 1 bis 4 zur Bekämpfung von Unkräutern.

8. Verfahren zur Herstellung von herbiziden Mitteln, dadurch gekennzeichnet, daß man Phenoxypropionsäure-Derivate der Formel (I) gemäß den Ansprüchen 1 bis 4 mit Streckmitteln und/oder oberflächenaktiven Stoffen vermischt.

9. Hydrochinonether der Formel

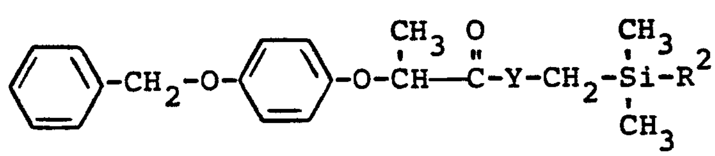 (XIII)

in welcher

Y für Sauerstoff, Schwefel oder die Gruppierung der Formel NR steht, worin R für Wasserstoff oder Alkyl mit 1 bis 6 Kohlenstoffatomen steht, und

$R^2$ für Alkyl mit 1 bis 6 Kohlenstoffatomen steht.

10. Verfahren zur Herstellung von Hydrochinonethern der Formel

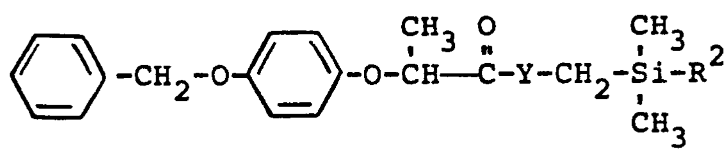

(XIII)

in welcher

Y für Sauerstoff, Schwefel oder die Gruppierung der Formel NR steht, worin

R für Wasserstoff oder Alkyl mit 1 bis 6 Kohlenstoffatomen steht, und

$R^2$ für Alkyl mit 1 bis 6 Kohlenstoffatomen steht,

dadurch gekennzeichnet, daß man Hydrochinon-monobenzylether der Formel

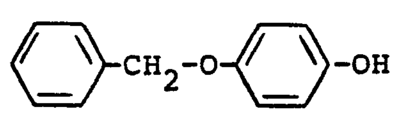

(XI)

mit Silylverbindungen der Formel

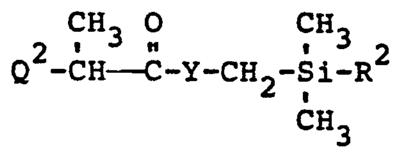 (XII)

in welcher

$R^2$ und Y die oben angegebene Bedeutung haben und

$Q^2$ für Chlor, Brom, Tosylat oder Mesylat steht,

gegebenenfalls in Gegenwart eines Säurebindemittels sowie gegebenenfalls in Gegenwart eines polaren Verdünnungsmittels umsetzt.